# EUROPEAN PATENT APPLICATION

(11) **EP 1 166 790 A1**
(43) Date of publication of application: **02.01.2002**
(21) Application number: 01115510.8
(22) Date of filing: 27.06.2001
(51) Int. Cl.: A61K 35/78, A23L 1/30, A23L 2/38

(54) **Sugar decomposition inhibitor, digestive enzyme activity inhibitor, insulin secretion controller, and healthy food and beverage**

(30) Priority: 28.06.2000 JP 2000194068
(71) Applicant: Ito En, Ltd., Tokyo 151-8550 (JP)
(72) Inventor: Suzuki, Yuko, Haibara-gun, Shizuoka 421-0516 (JP); Hayashi, Kazuhiko, Haibara-gun, Shizuoka 421-0516 (JP); Sakane, Iwao, Haibara-gun, Shizuoka 421-0516 (JP); Kakuda, Takami, Haibara-gun, Shizuoka 421-0516 (JP)
(74) Representative: Liedl, Christine, Dipl.-Chem.

(57) **Abstract**

A sugar decomposition inhibitor, a maltase activity inhibitor, a glucoamylase activity inhibitor, a sucrase activity inhibitor and an isomaltase activity inhibitor comprising as an effective component a hot-water extract of banaba, a synthetic resin adsorption fraction of said hot-water extract, or a mixture thereof are provided. Further, an insulin secretion controller comprising as an effective component a hot-water extract of banaba is also provided. In addition, a healthy food and beverage comprising a hot-water extract of banaba, a synthetic resin adsorption fraction of said hot-water extract or a mixture thereof and sugar except for monosaccharides are provided.

## Description

### BACKGROUND OF THE INVENTION

### FIELD OF THE INVENTION

The present invention relates to a sugar decomposition inhibitor, a digestive enzyme activity inhibitor, and an insulin secretion controller derived from banaba belonging to the family *Lythraceae* in the order *Myrtaceae,* and a healthy food and beverage that contains them.

### RELATED ART STATEMENT

Banaba (*Lagerstroemia speciosa L*. *Pers*.) belongs to the family *Lythraceae* in the order *Myrtaceae* and is a kind of *Lagerstroemia* distributed in the Tropical Asia. Juice obtained by boiling its leaves and flowers has been drunk from the old times as a treatment medicine of diabetes in the Philippines and other countries. Recently, in Japan, its effects of treating diabetes and controlling the blood sugar level are also perceived, and the number of people who drink its component as health tea or the like is increasing.

Relating to such pharmacological effects of banaba, it was already reported in the 1940's that the blood sugar level could be lowered by about 16-49 mg/dl with the dose of 1-2 g of dry leaf/kg of weight as a result of administering decoction of banaba dry leaves to normal domestic rabbits (F. Garcia: 'On the hypoglycemic effect of decoction of *Lagerstroemia speciosa* (Banaba)' J. Philip. Med. Assoc. 20, 395 (1940)).

Further, Japanese Non-examined Patent Publication No. 310,587/1993 discloses that the blood sugar level could be controlled significantly compared with the reference group that was not administered with banaba extract powder, as a result of breeding II-type diabetes mice with a feed containing 5% of banaba extract powder for three weeks after breeding them with one containing 3% of the same for a week. Furthermore, Japanese Non-examined Patent Publication No. 227,398/1997 discloses that effects of inhibiting α -amylase and lipase were confirmed and thus banaba is useful for treating and preventing fatness, diabetes, hyperlipemia, arteriosclerosis, blackhead, dermatitis and the like, as a result of measuring the absorbances AT, AS and AB after *in vitro* mixing a sample, which was obtained by adding 50% ethanol to pulverized dry banaba leaves at an amount corresponding to 100 times the weight of the leaves, filtering the extracted liquid obtained after heating and refluxing for one hour, and condensing the filtrate obtained, with α-amylase derived from human saliva, α -amylase or α -lipase derived from pig pancreas, or lipase derived from *Candida cylindraccae.*

As for the main action mechanism lowering the blood sugar level by banaba, it has been considered that banaba controls the increase in the blood sugar level by promoting intake of glucose from blood into cell (namely 'the promotion activity') (See 'Shokuhin To Kaihatsu (Food and Development, Japan)' Vol. 34, No. 8 (1999)).

Since it was confirmed that banaba also has an action inhibiting the enzyme activity for α -amylase which is the digestive enzyme of starch ('Nippon Nogeikagaku Kaishi (Journal of Japan Society for Bioscience, Biotechnology, and Agrochemistry)', Mar., 1997, 71), the inventors expected that there might be some new mechanisms lowering the blood sugar level by banaba.

### SUMMARY OF THE INVENTION

The inventors in this invention have obtained such result of the examination, clarifying the action mechanism lowering the blood sugar level by banaba, that the blood sugar level is restrained by administering the banaba extract simultaneously with starch which is a polysaccharide, but not restrained by administering it simultaneously with glucose which is a monosaccharide, and thus found out that the main action mechanism lowering the blood sugar level by banaba lies in the inhibition of sugar decomposition.

In this aspect, the present invention is about a sugar decomposition inhibitor comprising as an effective component a hot-water extract of banaba, a synthetic resin adsorption fraction of said hot-water extract of banaba or a mixture thereof.

In the above-mentioned examination, the inventors have newly found that the banaba extract has actions which inhibit the maltase activity, glucoamylase activity, sucrase activity and isomaltase activity, and also has an excellent effect of controlling the secretion of insulin.

In these aspects, the present invention provides a maltase activity inhibitor, glucoamylase activity inhibitor, sucrase activity inhibitor and isomaltase activity inhibitor respectively, comprising as an effective component a hot-water extract of banaba, a synthetic resin adsorption fraction of said hot-water extract of banaba or a mixture thereof.

In a preferred embodiment, the present invention provides a healthy food and beverage adding a hot-water extract of banaba, a synthetic resin adsorption fraction of said hot-water extract of banaba or a mixtute thereof, and sugar except for monosaccharides to food and beverages.

In the present invention, a 'hot-water extract of banaba' is obtained by extracting banaba with hot water, and a 'synthetic resin adsorption fraction" is an adsorption fraction obtained by adsorbing said hot-water extract of banaba with a styrene-divinylbenzene series synthetic resin or dextran series synthetic resin.

### BRIEF DESCRIPTION OF THE DRAWINGS

Figure 1 is a graph showing the influence of HWE (hot-water extract of banaba) to the blood sugar level at the time of starch administration.
Figure 2 is a graph showing the influence of HWE to the blood plasma insulin value at the time of starch administration.
Figure 3 is a graph showing the influence of HPWE (fraction of substances not adsorbed on the styrene-divinylbenzene series synthetic resin) and HPME (fraction of substances adsorbed on the styrene-divinylbenzene series synthetic resin) to the blood sugar level at the time of starch administration.
Figure 4 is a graph showing the influence of HPWE and HPME to the blood plasma insulin value at the time of starch administration.
Figure 5 is a graph showing the influence to the blood sugar level in the case that glucose and HWE are administered simultaneously.

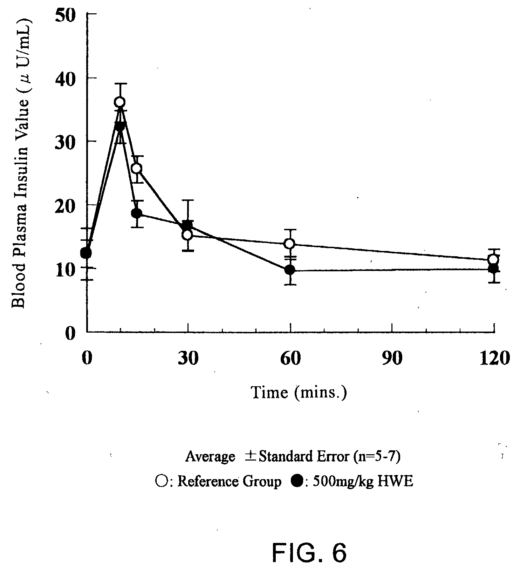

Figure 6 is a graph showing the influence to the blood plasma insulin value in the case that glucose and HWE are administered simultaneously.
Figure 7 is a graph showing the influence of HWE to the activities of α -amylase, maltase, glucoamylase, sucrase and isomaltase.
Figure 8 is a graph showing the influences of HWE, HPWE and HPME to the activity of glucoamylase.

### DETAILED DESCRIPTION OF THE INVENTION

The sugar decomposition inhibitor of the present invention is characterized by comprising an effective amount of a hot-water extract of banaba, a synthetic resin adsorption fraction of said hot-water extract of banaba or a mixture thereof.

This sugar decomposition inhibitor can restrain the rapid increase in the blood sugar level after a meal because it hinders digestion and absorption of sugar such as starch, glycogen, oligosaccharide, sucrose and maltose by inhibiting the decomposition of such sugar. Besides, the sugar decomposition inhibitor of the present invention acts effectively even in the case that it is taken at the same time at a meal, whereas most of the conventional sugar decomposition inhibitors were necessary to be taken before a meal. Accordingly, we expect that the sugar decomposition inhibitor of the present invention also acts effectively in the case of taking it together with a food or adding it to a food, and it demonstrates such an effect in treating diabetes and preventing fatness. Further, we expect it demonstrating in treating and preventing the heart tract system diseases such as myocardial infarction, arteriosclerosis and hypertension, the skin system diseases such as blackheads, pimples and other disease inflammations which are caused by supernutrition. Moreover, banaba is free from uneasiness in the feature of safety, as it has been used habitually from the old times in the Philippines and other countries.

The maltase activity inhibitor of the present invention is characterized by comprising an effective amount of a hot-water extract of banaba, a synthetic resin adsorption fraction of said hot-water extract of banaba or a mixture thereof.

This maltase activity inhibitor can restrain the increase in the blood sugar level because it inhibits the activity of maltase (α-glucosidase), which is an enzyme that hydrolyzes maltotriose and maltose formed by α -amylase decomposition of starch, so that maltotriose and maltose are not decomposed and thus glucose is restrained to form.

The glucoamylase activity inhibitor of the present invention is characterized by comprising an effective amount of a hot-water extract of banaba, a synthetic resin adsorption fraction of said hot-water extract of banaba or a mixture thereof.

This glucoamylase activity inhibitor can restrain the increase in the blood sugar level because it inhibits the activity of glucoamylase, which is an enzyme that decomposes oligosaccharides mainly consisting of four to nine bonds of glucose unit by cutting the α -1 and α -4 bonds thereof, so that oligosaccharides are not decomposed and thus glucose is restrained to form.

Maltase and glucoamylase described above also contribute to decomposing amylose which is composed of glucose units combined one another at the α - 1 and α - 4 bonds in a straight chain, as well as cut the α - 1 and α -4 bonds of oligosaccharides. Therefore, the maltase activity inhibitor and glucoamylase activity inhibitor of the present invention are effective, especially in the case that they are taken simultaneously with a food containing much amylose, so that a dieting food or beverage can be provided by adding them to a food containing much amylose.

The isomaltase activity inhibitor of the present invention is characterized by comprising an effective amount of a hot-water extract of banaba, a synthetic resin adsorption fraction of said hot-water extract of banaba or a mixture thereof.

This isomaltase activity inhibitor can restrain the increase in the blood sugar level because it inhibits the activity of isomaltase, which is an enzyme that decomposes the limited dextrins, formed by α -amylase decomposition of starch, by cutting the α -1 and α - 6 bonds thereof, so that the limited dextrin is not decomposed and thus glucose is restrained to form.

Isomaltase described above also contributes to the decomposition of amylopectin which is composed of many branches combined to the straight chain of amylose at the α - 1 and α - 6 bonds thereof, as well as it cuts the α - 1 and α -6 bonds of the limit dextrin. Therefore, the isomaltase activity inhibitor of the present invention are effective, particularly in the case that it is taken simultaneously with a food containing much amylopectin, so that a dieting food or beverage can be provided by adding it to a food containing much amylopectin.

The amylose content of starch is usually 20 to 30% by weight and the residual is amylopectin. The starches of glutinous rice and corn do not contain amylose at all while the starches of nonglutinous rice, fermented soybean and green gram contain 20%, 35% and 35% amylose, respectively.

The sucrase activity inhibitor of the present invention is characterized by comprising an effective amount of a hot-water extract of banaba, a synthetic resin adsorption fraction of said hot-water extract of banaba or a mixture thereof.

This sucrase activity inhibitor can restrain the increase in the blood sugar level because it inhibits the activity of sucrase, which is an enzyme that decomposes sucrose (cane sugar), so that sucrose is not decomposed and thus monosaccharides are restrained to form. Therefore, the sucrase activity inhibitor of the present invention is effective, especially in the case that it is taken simultaneously with a food containing much sucrose, so that a dieting food or beverage can be provided by adding it to a food containing much sucrose.

The insulin secretion controller of the present invention is characterized by comprising an effective amount of a hot-water extract of banaba obtained by extracting banaba with hot water.

Furthermore, since the secretion of a large quantity of insulin stimulated by supernutrition and the like induces an unbalance of the metabolism and degradation of the sugar-resistant function, various diseases such as diabetes and hyperlipemia are caused, and the development of a substance which can effectively control the secretion of insulin has been desired since before. The insulin secretion controller of the present invention can be exactly fitted for such needs and thus can control such secretion of a large quantity of insulin, especially the rapid secretion of insulin after a meal.

With the above-mentioned effects of the banaba extract, the present invention also provides the healthy food and beverage, comprising a hot-water extract of banaba, a synthetic resin adsorption fraction of said hot-water extract of banaba or a mixture thereof, and sugar except for monosaccharides.

Said sugar except for monosaccharides can be defined as a sugar consisting of at least two monosaccharides, for example, sucrose, maltose, dextrin and glycogen.

The healthy food and beverage of the present invention is strikingly effective above all as a dieting food or beverage or as a food or beverage for improving the eating habits in diabetes cases and the like, because they do not contain monosaccharides, which is absorbed into human body as it is without decomposing, and do contain sugar alone which is only absorbed after being decomposed, so that they can hinder digestion and absorption of a food or beverage even if it contains much sugar.

### DESCRIPTION OF THE PREFERRED EMBODIMENT

The preferred embodiment of the present invention is described hereinafter.

### (The Sugar Decomposition Inhibitor and the Digestive Enzyme Activity Inhibitors of Every Kind)

The sugar decomposition inhibitor, maltase activity inhibitor, glucoamylase activity inhibitor, sucrase activity inhibitor, isomaltase activity inhibitor and insulin secretion controller of the present invention can be prepared by containing the hot-water extract of banaba, the synthetic resin adsorption fraction of the hot-water extract of banaba or the mixture thereof, which is called inclusively the 'banaba extract' in the present invention.

'Banaba' in the present invention means each part of plant bodies of leaves, flowers, stems, xylem, roots and fruits obtained from *Lagerstroemia speciosa L. Pers.* which belongs to the family *Lythraceae* in the order *Myrtaceae,* or a mixture of at least two selected from the each part of the plant bodies.

This banaba is necessary to be sufficiently sterilized by heating, for example, with an autoclave before the extraction treatment described below, because it contains a great deal of sundry bacteria. In particular, as described in Japanese Patent Publication No. 2,818,458, it is preferred that dried and crushed banaba leaves are sterilized by heating, kept under a bacterial culture environment, and further sterilized by heating.

The above-mentioned 'hot-water extract of banaba' can be obtained by extracting the aforementioned banaba with hot water. Specifically, it can be obtained as extracted liquid, diluted or concentrated liquid, essence or dry material of said extracted liquid by extracting the dried and crushed banaba leaves with hot water at a temperature from about 60 to 100°C, more preferably at a temperature from 95 to 100°C which is almost or that of the boiling point.

The above-mentioned 'synthetic resin adsorption fraction' of banaba is the component adsorbed to the styrene-divinylbenzene series synthetic resin or dextran series synthetic resin. Specifically, it can be obtained as eluate, diluted or concentrated liquid, essence or dry material of said eluate by dissolving the above-mentioned hot-water extract of banaba in distilled water, applying the solution to a column packed with the styrene-divinylbenzene series synthetic resin or dextran series synthetic resin, subsequently washing the column with distilled water, and then, eluting the adsorbed substances with 100% of methanol.

As the styrene-divinylbenzene series synthetic resin, Diaion HP-10, 20, 30, 40 and 50 produced by Mitsubishi Kasei Kogyo K.K., Amberlite XAD-2 and 4 produced by Organo Corp., Duolite S series produced by Sumitomo Chemical Co., Ltd. and the like can be used. As the dextran series synthetic resin, Sephadex LH-20 produced by Pharmacia can be used. The dextran series synthetic resin forms a polysaccharide-like network in which the constituent dextran chains are cross-linked three-dimensionally, so that its ratio of the number of carbon atoms to that of hydroxyl radicals is larger, particularly for LH-20 which has hydroxypropyl radicals ether-linked in each glucose unit of the dextran chain. Therefore, the dextran series synthetic resin (gel), especially LH-20, has both properties of hydrophilicity and lipophilicity, and is considered similar to the styrene-divinylbenzene series synthetic resin concerning the adsorptive property. From the viewpoint of financial aspect, however, the styrene-divinylbenzene series synthetic resin is better used at present.

The above-mentioned hot-water extract of banaba and synthetic resin adsorption fraction of banaba can also be used in the mixture thereof as the effective component, while they can be used respectively and solely as the effective component of the present invention.

It is also possible to raise synergistically the effect of the effective components by combining them to contain with the conventional activity inhibitors for digestive enzymes, such as protein substances extracted from wheat (M. D. O'Donnell et al., Biochemi. Biophys. Acta 422 (1976) 159-169), polysaccharides derived from soybeans (Japanese Non-examined Patent Publication No. 290,187/1991), protein substances NSAI-I and NSAI-II extracted from *Colocasia esculenta* (Japanese Patent Application No. 95,992/1990) and crude essence extracted from *Laurus nobilis L.* (Japanese Patent Application No. 130,852/1990), so that all the effective components of the present invention can be derived from natural substances as well as the digestive enzyme activity effect thereof can be raised synergistically.

The sugar decomposition inhibitor, maltase activity inhibitor, glucoamylase activity inhibitor, sucrase activity inhibitor, isomaltase activity inhibitor and insulin secretion controller can be provided as medicines, quasi drugs, foods and beverages having medical effects such as healthy foods and healthy beverages, food additives, feeds and feed additives.

As for the forms, the products of the present invention can be provided as dry powders by freeze drying, spray drying, or the like, and also, as solutions, tablets, granules, dragees, soft and hard capsules, suspensions, emulsions, ampules, injections, and the like. When, for example, the products of the present invention are prepared as quasi drugs and provided in the forms of bottled drinks or the like, or tablets, capsules, granules or the like which are easily taken, more sufficient pharmacological effects thereof to living body can be expected because it is usually easier to be taken.

Besides, in making the medicines or drugs, the products of the present invention can be optionally made to various dosage forms, according to the conventional methods, by adding surfactants, vehicles, taste correctives, smell correctives, lubricants, adjuvant drugs or the like, or by transforming into aqueous solutions or oily substances. Moreover, the effects of medicines can also be maintained for a long term by coating the surface thereof in the making.

The dosage of the products of the present invention as a medicine, which should be properly changed in accordance with the method of administration, seriousness of disease, age, and the like, is preferable in the range of 50 to 5,000 mg/day for adults and in the range of 15 to 1,500 mg/day for children, and the concentration of the effective component as a medicine is preferable in the range of 0.1 to 100% by weight, more preferably in the range of 1 to 50% by weight.

The dosage of the maltase activity inhibitor is preferable in the range of 100 to 1,000 mg/day for adults and in the range of 35 to 350 mg/day for children; the dosage of the glucoamylase activity inhibitor is preferable in the range of 100 to 1,000 mg/day for adults and in the range of 35 to 350 mg/day for children; the dosage of the sucrase activity inhibitor is preferable in the range of 300 to 3,000 mg/day for adults and in the range of 100 to 1,000 mg/day for children; and the dosage of the isomaltase activity inhibitor is preferable in the range of 500 to 5,000 mg/day for adults and in the range of 150 to 1,500 mg/day for children. Further, the dosage of the insulin secretion controller is preferable in the range of 100 to 1,000 mg/day for adults and in the range of 35 to 350 mg/day for children.

The sugar decomposition inhibitor, maltase activity, glucoamylase activity inhibitor, sucrase activity inhibitor, isomaltase activity inhibitor and insulin secretion controller of the present invention may of course be taken before a meal for preventing worries and diseases caused by supernutrition such as fatness and diabetes, and also, at the same time as a meal or by mixing into a food. Thus, one of the features of the present invention is that the rapid increase in the blood sugar level after a meal can be effectively restrained even in the case that the products of the present invention are taken with a food. Above all, in the case that the insulin secretioncontrollercomprisingthehot-waterextract of banaba as the effective component is taken with a food, the effect thereof is remarkable.

### (Healthy Foods and Beverages)

The healthy food and beverage of the present invention can be prepared by containing the hot-water extract of banaba, synthetic resin adsorption fraction of the hot-water extract of banaba or the mixtute thereof, and the sugar except for monosaccharides.

As the hot-water extract of banaba and synthetic resin adsorption fraction, the same materials as described above can be used. Further, the necessity of sterilizing banaba also is the same as described above.

The monosaccharides include glucose, fructose, galactose, and the like, and the sugar except for monosaccharides includes sucrose, maltose, dextrin, glycogen, and the like.

In this case, the sugar except for monosaccharides can also be blended with a mixture composed of at least two kinds of such sugar, while it can be used solely. Moreover, it can be blended as foods or beverages containing these kinds of sugar, for example, such beverages as vegetable or fruit juice, tea, milk products, lactic beverages, alcoholic drinks, processed marine products, milk products, such cooked foods as curry and stew, confectionaries, bread, ice cream and the like from which monosaccharides are removed.

Furthermore, the healthy food and beverage of the present invention can also be prepared by removing monosaccharides from any food or beverage and mixing with the above-mentioned banaba extract.

In preparing the healthy food or beverage, it maybe optionally mixed, according to the conventional methods, with various proteins, vitamins, minerals, and the like, which are base materials and foods permitted in manufacturing foods and beverages, such as extenders, vehicles, carbonic acid, pelletizing materials, diluents, sweet materials, flavors, oil,fats, and the like.

The concentration of the effective component in the healthy food and beverage is preferable in the range of 0.05 to 5% by weight.

The healthy food and beverage of the present invention are strikingly effective as a dieting food, or a healthy food or beverage for improving the eating habits of diabetes patients and the like, because they can control digestion and absorption of such sugar even in the case of foods or beverages containing much sugar. As the dieting food above all, they can restrain decomposition and absorption of sugar which occupies most of the total energy taken, therefore, a significant effect is expected on the point that dieting can be gradually performed maintaining health without excessive dieting, such as fasting.

Details in the present invention are explained hereinafter on the basis of various examinations.

### (Preparation of Examination Materials)

Appropriate raw banaba leaves harvested in the Philippines were sufficiently dried naturally or forcedly, ground by a crusher, and 1 kg of ground and well-mixed banaba leaves was extracted with about 50 l of distilled water at a temperature from 95 to 100°C for about 30 minutes. The extracted solution thus obtained was filtered, centrifuged, and then, concentrated by an evaporator, followed by freeze drying or spray drying to produce a hot-water extract of banaba (hereinafter referred to 'HWE').

Next, a glass column having a diameter of 10 cm and a length of 100 cm was packed with 2.5 l of styrene-divinylbenzene series synthetic resin (Diaion HP-20, Mitsubishi Kasei Kogyo K.K.), and the packed resin was washed with methanol, and then, distilled water. Subsequently, the above-mentioned HWE was dissolved in 1l of distilled water and applied to the glass column. Then, distilled water was applied at an amount corresponding to about six times the volume of the column to remove a fraction of substances not adsorbed on the styrene-divinylbenzene series synthetic resin (hereinafter referred to 'HPWE'), and after that, 100% methanol at an amount corresponding to about five times the volume of the column was applied to elute a fraction of substances adsorbed on the styrene-divinylbenzene series synthetic resin (hereinafter referred to 'HPME').

The HPWE and HPME thus obtained were concentrated and dried by the method of freeze drying, spray drying, or the like to obtain each dried material. The yield of HWE, HPWE and HPME to the weight of dried leaves was 17%, 9% and 8% by weight, respectively, and the polyphenol content of them determined by the Folin-Denis method was 59%, 28% and 80% by weight, respectively.

### (Sugar Loading Examination in Rats)

A soluble dextrin or glucose as sugar was used at 1 g/kg.

Wistar-type male rats whose age was 8 weeks and which had fasted for 18 hours were administered by mouth with a mixture of a sugar solution and a solution of hot-water extract of banaba having various concentrations. Before and at the time of 10, 15, 30, 60, 120 minutes after the administration, Blood-collecting was conducted from orbit vein bush of the rats under ether anesthesia, and the blood collected was promptly centrifuged to measure the blood sugar level and insulin concentration in blood plasma.

### (Measurement of various glucosidase inhibition actions)

An acetone powder of rat small intestine (produced by Sigma) was added to 56 mM maleic acid buffer solution (pH 6.0) at an amount corresponding to nine times the weight of said acetone powder, and the mixture obtained was homogenized by using a Polytron homogenizer and cooling with ice, and then centrifuged (3,000 rpm, 10 mins. with 4°C), and the supernatant liquid obtained as a crude enzyme solution was examined on the enzyme activities of glucoamylase, maltase, sucrase and isomaltase. In the examination, the crude enzyme solution was used after diluting to four times for glucoamylase, twenty times for maltase, and two times for sucrase and isomaltase, respectively. As the substrate, 2% soluble starch solution for the glucoamylase reaction, 2% maltose for the maltase reaction, 2% sucrose for the sucrase solution and 2% palatinose for the isomaltase reaction were used, respectively.

The measurement of the inhibiting actions were conducted by adding 50 *µ* l of the crude enzyme solution to the various 2% substrate solutions (HWE, HPWE and HPME), which were prepared by dissolving into the above- mentioned buffer solution, further adding 50 *µ* l of solution of banaba leaves extract, and allowing to react at 37°C. After heating the obtained product in boiled water to cease the enzyme reaction, the resulting glucose was determined by the HK-G6PD method. The inhibiting action of banaba leaves extract was indicated by the residual activity which was defined as 100% of the amount of glucose formed when no inhibitor was added as reference.

### (Statistical Treatment)

The significant difference test for mean values between two groups was conducted by using Student's t-test, and that for mean values among many groups was done by Duncan's multiple-range test (p<0.05).

### (Investigation on the Inhibiting Action of Blood Sugar Increase after Starch Loading)

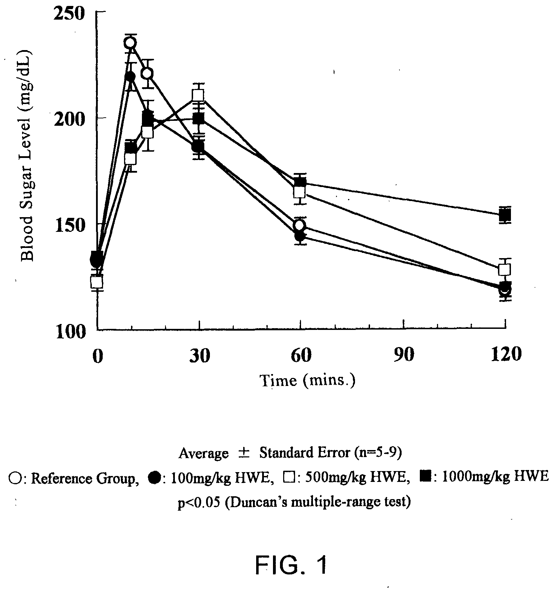

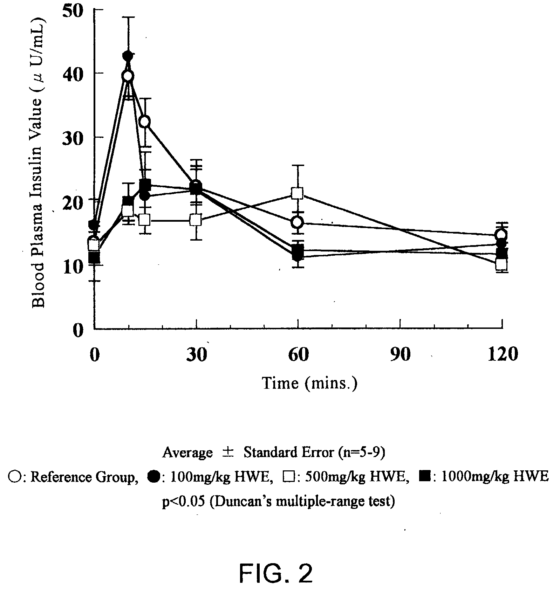

The results of administering HWE to the rats at the same time with 1 g/kg of soluble starch are shown in Figures 1 and 2. The blood sugar level of the reference group reached a maximum after 10 minutes, and then, decreased with the elapse of time, and returned almost to the level before administering starch after 120 minutes (Figure 1). On the other hand, in the case of administering HWE at the rate of 100, 500 or 1,000 mg/kg, the blood sugar level after 10 minutes decreased depending on the concentration significantly compared with that of the reference group, while the insulin value of blood plasma reached a maximum at the time of 10 minutes after administering when the blood sugar level of the reference group indicated a maximum. The insulin value of blood plasma for the group of 100 mg/kg HWE indicated a maximum at the time of 10 minutes after administration as well as the reference group, but it was restrained significantly after 15 minutes. For the group of 500 mg/kg HWE, any rapid increase in the insulin value was not observed after administering, and the insulin value was restrained significantly at the times of 10 and 15 minutes after administering compared with the reference group (Figure 2).

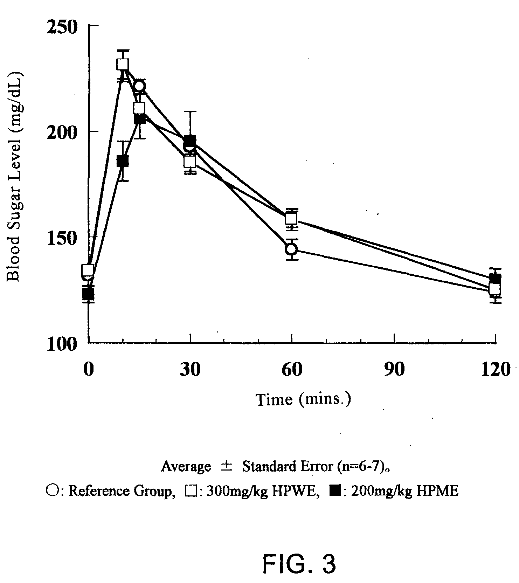

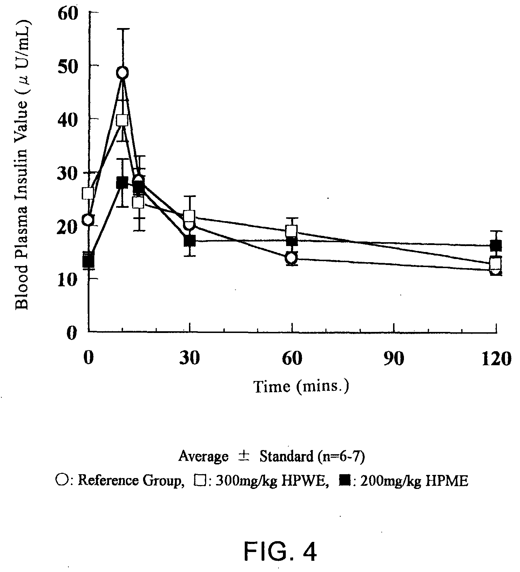

Next, in the starch loading examination, as a result of investigating the influence of HPWE or HPME obtained by fractionating HWE with HP-20 to the blood sugar level and insulin value of blood plasma, the blood sugar level for HPME decreased significantly compared with that of the reference group at the time of 10 minutes after administering, while any significant difference for HPWE was not observed (Figure 3). The insulin value of blood plasma did not indicate any significant difference for each group of HPWE nor HPME, but a tendency of restraining the increase in the value for HPME was observed after 10 minutes (Figure 4).

Furthermore, the insulin value could be remarkably restrained by administering HWE, which is a distinguishing effect compared with the restraining effect of the blood sugar level, while the reason is not yet clarified.

### (Investigation on the Restraining Effect of the Blood Sugar Level after Glucose Loading)

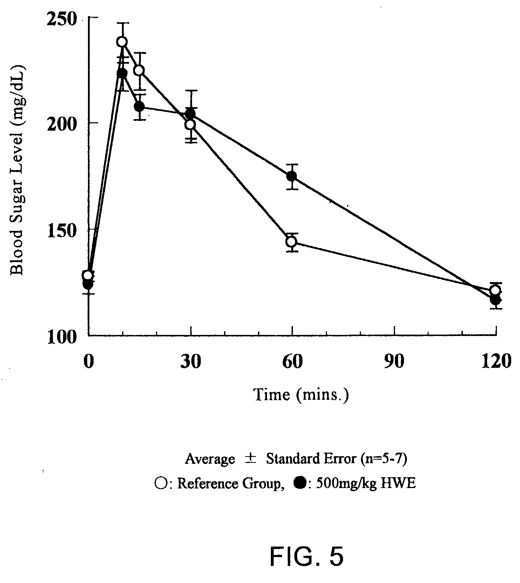

As a result of administering to rats glucose and HWE at the rate of 500 mg/kg at the same time, the blood sugar level did not show any significant difference at the time of 10 minutes after administering, but indicated a significant higher value after 60 minutes compared with that of the reference group (Figure 5) . In addition, the insulin value of blood plasma as well did not show any significant difference after 10 minutes.

### (The Inhibiting Action of Sugar Digestive Enzymes by Banaba)

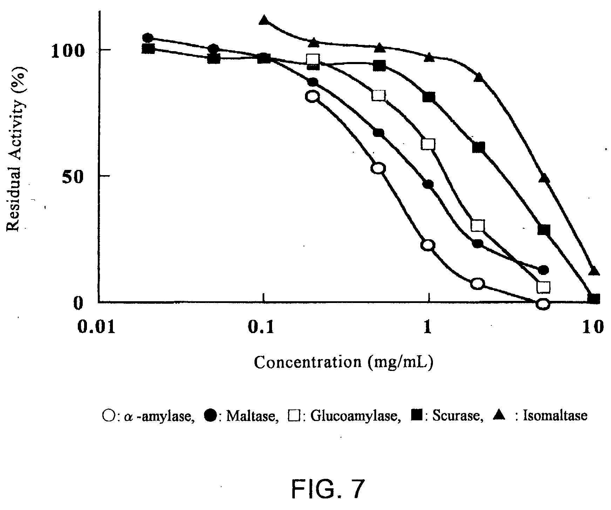

The inhibiting action of HWE to the activations of maltase, glucoamylase, sucrase and isomaltase derived from rat small intestine is shown in Figure 7.

The HWE showed such inhibiting action to all the enzymes examined, the IC₅₀ values for them were 0.89, 1.24, 2.85 and 4.95 mg/ml, respectively.

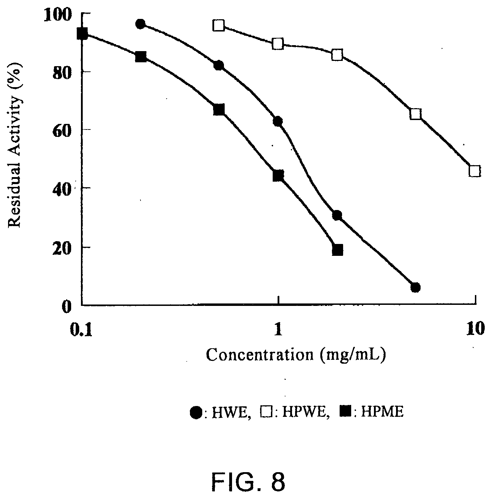

Moreover, the inhibiting action of HWE, HPWE and HPME to amylase is shown in Figure 8. The IC₅₀ values for them were 1.24, 8.60 and 0.83 mg/ml, respectively.

Both of HWE and HPME of the banaba leaves extract used in this examination lowered the blood sugar level significantly at the time of 10 minutes after administering by adding them at the same time with starch, so that the preventive effect of them to diabetes and fatness can be sufficiently expected by controlling the rapid increase in the blood sugar level after a meal.

Furthermore, considering that, while HWE and HPME inhibited *in vitro* the activities of the sugar digestive enzymes, HWE did not restrain the increase in the blood sugar level by administering it at the same time with glucose, the restraint of increase in the blood sugar level after a meal can be supposed due to restraint of sugar decomposition, which may be one of the action mechanisms of such anti-diabetes agent.

### (Example 1)

A hot water extract of banaba was dissolved in 2,000 ml of hot water at 95°C, and was filtered and cooled, further was filtered with flannel, and then, was added with Vitamin C and sodium bicarbonate. Subsequently, the prepared solution obtained was heated again to 95°C, and was packed into a can and sterilized with a retort to obtain a sugar decomposition inhibitor (which can also be used as amaltase activation inhibitor, glucoamylase activity inhibitor or insulin secretion controller) having the following combination:

| | |
|---|---|
| Total volume··· | 200 ml |
| Hot water extract of banaba··· | 100 mg |
| Vitamin C··· | 40 mg |
| Sodium bicarbonate··· | 40 mg |
| Spice··· | proper amount |
| Water··· | balance |

### (Example 2)

A hot water extract of banaba was dissolved in 2,000 ml of hot water at 95°C, and was filtered and cooled, further was filtered with flannel, and then, was added with Vitamin C and sodium bicarbonate. Subsequently, the prepared solution obtained was heated again to 95°C, and was packed into a can and sterilized with a retort to obtain a sucrase activity inhibitor having the following combination:

| | |
|---|---|
| Total volume··· | 200 ml |
| Hot water extract of banaba··· | 300 mg |
| Vitamin C··· | 40 mg |
| Sodium bicarbonate··· | 40 mg |
| Spice··· | proper amount |
| Water··· | balance |

### (Example 3)

A hot water extract of banaba was dissolved in 2,000 ml of hot water at 95°C, and was filtered and cooled, further was filtered with flannel, and then, was added with Vitamin C and sodium bicarbonate. Subsequently, the prepared solution obtained was heated again to 95°C, and was packed into a can and sterilized with a retort to obtain a isomaltase activity inhibitor having the following combination:

| | |
|---|---|
| Total volume··· | 200 ml |
| Hot water extract of banaba··· | 500 mg |
| Vitamin C··· | 40 mg |
| Sodium bicarbonate··· | 40 mg |
| Spice··· | proper amount |
| Water··· | balance |

### (Example 4)

A healthy food (namely 'furikake' eatenwith rice, which is like the spread on bread) having the following combination was prepared:

| | |
|---|---|
| Total weight··· | 3 g |
| Hot water extract of banaba··· | 500 mg |
| Cut sheet of seaweed··· | 1,500 mg |
| Egg particle··· | 400 mg |
| Roasted sesame··· | 250 mg |
| Salt··· | 240 mg |
| Shaved meat of dried bonito··· | 200 mg |
| Seasoning (amino acid etc.)··· | proper amount |

### (Example 5)

A healthy beverage (juice) containing a sucrase activity inhibitor was prepared, which has the following combination:

| | |
|---|---|
| Total volume··· | 200 ml |
| Hot water extract of banaba··· | 500 mg |
| Fruit juice of Japanese orange··· | 20 g |
| Sucrose··· | 20 g |
| Citric acid··· | 2 g |
| Vitamin C··· | proper amount |
| Spice··· | proper amount |
| Water··· | balance |

## Claims

1. A sugar decomposition inhibitor comprising an effective amount of a hot-water extract of banaba obtained by extracting banaba with hot water.

2. The sugar decomposition inhibitor as claimed in claim 1, wherein comprising an effective amount of a mixture of said hot-water extract of banaba and a synthetic resin adsorption fraction of banaba obtained by adsorbing said hot-water extract of banaba with a styrene-divinylbenzene series synthetic resin or dextran series synthetic resin.

3. A sugar decomposition inhibitor comprising an effective amount of a synthetic resin adsorption fraction of banaba, obtained by adsorbing a hot-water extract of banaba obtained by extracting banaba with hot water, with a styrene-divinylbenzene series synthetic resin or dextran series synthetic resin.

4. A maltase activity inhibitor comprising an effective amount of a hot-water extract of banaba obtained by extracting banaba with hot water.

5. The maltase activity inhibitor as claimed in claim 4, wherein comprising an effective amount of a mixture of said hot-water extract of banaba and a synthetic resin adsorption fraction of banaba obtained by adsorbing said hot-water extract of banaba with a styrene-divinylbenzene series synthetic resin or dextran series synthetic resin.

6. A maltase activity inhibitor comprising an effective amount of a synthetic resin adsorption fraction of banaba, obtainedby adsorbing a hot-water extract of banaba obtained by extracting banaba with hot water, with a styrene-divinylbenzene series synthetic resin or dextran series synthetic resin.

7. A glucoamylase activity inhibitor comprising an effective amount of a hot-water extract of banaba obtained by extracting banaba with hot water.

8. The glucoamylase activity inhibitor as claimed in claim 7, wherein comprising an effective amount of a mixture of said hot-water extract of banaba and a synthetic resin adsorption fraction of banaba obtained by adsorbing said hot-water extract of banaba with a styrene-divinylbenzene series synthetic resin or dextran series synthetic resin.

9. A glucoamylase activity inhibitor comprising an effective amount of a synthetic resin adsorption fraction of banaba, obtained by adsorbing a hot-water extract of banaba obtained by extracting banaba with hot water, with a styrene-divinylbenzene series synthetic resin or dextran series synthetic resin.

10. A sucrase activity inhibitor comprising an effective amount of a hot-water extract of banaba obtained by extracting banaba with hot water.

11. The sucrase activity inhibitor as claimed in claim 10, wherein comprising an effective amount of a mixture of said hot-water extract of banaba and a synthetic resin adsorption fraction of banaba obtained by adsorbing said hot-water extract of banaba with a styrene-divinylbenzene series synthetic resin or dextran series synthetic resin.

12. A sucrase activity inhibitor comprising an effective amount of a synthetic resin adsorption fraction of banaba, obtained by adsorbing a hot-water extract of banaba obtained by extracting banaba with hot water, with a styrene-divinylbenzene series synthetic resin or dextran series synthetic resin.

13. An isomaltase activity inhibitor comprising an effective amount of a hot-water extract of banaba obtained by extracting banaba with hot water.

14. The isomaltase activity inhibitor as claimed in claim 13, wherein comprising an effective amount of a mixture of said hot-water extract of banaba and a synthetic resin adsorption fraction of banaba obtained by adsorbing said hot-water extract of banaba with a styrene-divinylbenzene series synthetic resin or dextran series synthetic resin.

15. An isomaltase activity inhibitor comprising an effective amount of a synthetic resin adsorption fraction of banaba, obtainedby adsorbing a hot-water extract of banaba obtained by extracting banaba with hot water, with a styrene-divinylbenzene series synthetic resin or dextran series synthetic resin.

16. An insulin secretion controller comprising an effective amount of a hot-water extract of banaba obtained by extracting banaba with hot water.

17. A healthy food and beverage produced by adding a hot-water extract of banaba obtained by extracting banaba with hot water, and sugar except for monosaccharides to food or beverage.

18. The healthy food and beverage as claimed in claim 17, produced by further adding a synthetic resin adsorption fraction of banaba obtained by adsorbing said hot-water extract of banaba with a styrene-divinylbenzene series synthetic resin or dextran series synthetic resin to food or beverage.

19. A healthy food and beverage comprising:
(a) a hot-water extract of banaba obtained by extracting banaba with hot water, and
(b) sugar except for monosaccharides.

20. The healthy food and beverage as claimed in claim 19, further comprising a synthetic resin adsorption fraction of banaba obtained by adsorbing said hot-water extract of banaba with a styrene-divinylbenzene series synthetic resin or dextran series synthetic resin.

21. A healthy food and beverage produced by adding a synthetic resin adsorption fraction of banaba obtained by adsorbing a hot-water extract of banaba, obtained by extracting banaba with hot water with a styrene-divinylbenzene series synthetic resin or dextran series synthetic resin, and sugar except for monosaccharides to food or beverage.

22. A healthy food and beverage comprising:
(a) a synthetic resin adsorption fraction of banaba, obtained by adsorbing a hot-water extract of banaba obtained by extracting banaba with hot water, with a styrene-divinylbenzene series synthetic resin or dextran series synthetic resin, and
(b) sugar except for monosaccharides.

23. The healthy food and beverage as claimed in one of claims 17-22, wherein said sugar except for monosaccharides is a sugar consisting of at least two monosaccharides.

24. The healthy food and beverage as claimed in one of claims 17-22, wherein said sugar except for monosaccharides is at least one member selected from the group consisting of sucrose, maltose, dextrin and glycogen.
